(19)
Europäisches Patentamt

European Patent Office

Office européen des brevets

(11) **EP 4 756 090 A1**

(12) **EUROPEAN PATENT APPLICATION**
published in accordance with Art. 153(4) EPC

(43) Date of publication:
**10.06.2026 Bulletin 2026/24**

(21) Application number: **24862630.1**

(22) Date of filing: **26.08.2024**

(51) International Patent Classification (IPC):
**D04H 1/425** (2012.01)    **A47K 7/00** (2006.01)
**A47L 13/17** (2006.01)

(52) Cooperative Patent Classification (CPC):
**A47K 7/00; A47L 13/17; D04H 1/425**

(86) International application number:
**PCT/JP2024/030175**

(87) International publication number:
**WO 2025/052978 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.09.2023 JP 2023145102**

(71) Applicant: **Unicharm Corporation
Shikokuchuo-shi, Ehime 799-0111 (JP)**

(72) Inventors:
• **IKEUCHI, Norihito
Kanonji-shi, Kagawa 769-1602 (JP)**
• **NOGUCHI, Atsushi
Kanonji-shi, Kagawa 769-1602 (JP)**
• **KIMURA, Akihiro
Kanonji-shi, Kagawa 769-1602 (JP)**

(74) Representative: **Dolleymores
9 Rickmansworth Road
Watford, Hertfordshire WD18 0JU (GB)**

(54) **NONWOVEN FABRIC FOR WET WIPES, WET WIPES, PACKAGE OF WET WIPES, AND USE OF WATER-REPELLENT CELLULOSE FIBERS**

(57)    The purpose of the present disclosure is to provide a nonwoven fabric for wet wipes, said wet wipes formed of said nonwoven fabric having superior properties allowing easy taking out from a package. More specifically, this nonwoven fabric for wet wipes is characterized in that it contains water-repellent cellulose fibers.

Fig. 1

EP 4 756 090 A1

**Description**

FIELD

The present disclosure relates to a nonwoven fabric for a wet wipe, a wet wipe, a package of wet wipes, and use of a water-repellent cellulose fiber.

BACKGROUND

In the packages for wet wipes, wet tissues, etc., and in particular, in pop-up-type packages therefor, when a single wet wipe is extracted from the package, in some cases, a plurality of wet wipes, including the next wet wipe, are simultaneously extracted (hereinafter, sometimes referred to as "multiple-wipe extraction tendency").
Studies for overcoming such a problem related to multiple-wipe extraction tendency are being performed.
For example, Patent Literature 1 discloses a wet wipe comprising a nonwoven fabric produced by a wet process and a chemical solution with which the nonwoven fabric is impregnated, wherein the nonwoven fabric comprises synthetic fiber having a specific gravity exceeding 1.0 in an amount of 5 to 40 mass% of the nonwoven fabric and cellulose fiber in an amount of 60 to 95 mass% of the nonwoven fabric, and the synthetic fiber comprises synthetic fiber having an irregular cross-sectional shape in an amount of 20 to 100 mass% of the synthetic fiber.
Patent Literature 2 discloses a wet wipe comprising a nonwoven fabric and a chemical solution with which the nonwoven fabric is impregnated, wherein the nonwoven fabric comprises hydrophobic fiber, and the chemical solution comprises (i) a cationic surfactant, (ii) a low HLB surfactant having an HLB of 3.0 to 7.0, and (iii) water.

[CITATION LIST]

[PATENT LITERATURE]

[PTL 1] Japanese Unexamined Patent Publication (Kokai) No. 2014-94188
[PTL 2] Japanese Unexamined Patent Publication (Kokai) No. 2022-92982

SUMMARY

[TECHNICAL PROBLEM]

Patent Literature 1 and Patent Literature 2 do not disclose the wet wipe according to the present disclosure.
An object of the present disclosure is to provide a nonwoven fabric for a wet wipe which provides excellent extractability of the wet wipe formed from the nonwoven fabric.

[SOLUTION TO PROBLEM]

The present inventors have discovered a nonwoven fabric for a wet wipe, wherein the nonwoven fabric comprises a water-repellent cellulose fiber.

[ADVANTAGEOUS EFFECTS OF INVENTION]

The nonwoven fabric for a wet wipe according to the present disclosure achieves excellent extractability of the wet wipe formed from the nonwoven fabric.

BRIEF DESCRIPTION OF DRAWINGS

FIG. 1 is a view detailing a package of wet wipes 1 according to a first embodiment.
FIG. 2 is an end view of the package of wet wipes 1 taken along line II-II.
FIG. 3 is a view detailing a plurality of wet wipes 9.
FIG. 4 is an end view of a package of wet wipes 1 according to a second embodiment.

DESCRIPTION OF EMBODIMENTS

Specifically, the present disclosure relates to the following Aspects.

[Aspect 1]

A nonwoven fabric for a wet wipe, wherein
the nonwoven fabric comprises a water-repellent cellulose fiber.
The nonwoven fabric comprises a water-repellent cellulose fiber. This water-repellent cellulose fiber is a cellulose fiber having water repellency and exhibits water repellency, but once wetted, can retain a certain amount of moisture within the fiber and has a certain water swelling degree.
The water-repellent cellulose fiber has high chemical solution retention properties and equivalent or higher water repellency, as compared to synthetic fiber, hydrophobic fiber, etc., which have hydrophobicity and do not retain moisture (hereinafter sometimes referred to as "hydrophobic fiber, etc."). Thus, the nonwoven fabric comprising a water-repellent cellulose fiber according to the present invention has (i) high chemical solution retention properties and (ii) equivalent or higher water repellency, as compared to a nonwoven fabric comprising hydrophobic fiber, etc., instead of the water-repellent cellulose fiber, due to the properties of the water-repellent cellulose fiber. As a result, in a wet wipe formed from a nonwoven fabric comprising the water-repellent cellulose fiber, adjacent wet wipe portions are less likely to adhere to each other and are more likely to separate, as compared to a wet wipe formed from a nonwoven fabric comprising hydrophobic fiber, etc., instead of the water-repellent cellulose fiber.
The water-repellent cellulose fiber has equivalent chemical solution retention properties and high water repellency, as compared to conventional cellulose fiber that has not been subjected to water repellency treatment (hereinafter, in order to distinguish from water-repellent cellulose fiber, "conventional cellulose fiber that has not been subjected to water repellency treatment" may be referred to as "hydrophilic cellulose fiber"). Thus, the nonwoven fabric comprising a water-repellent cellulose fiber according to the present invention has (i) equivalent chemical solution retention properties and (ii) high water repellency, as compared to a nonwoven fabric comprising hydrophilic cellulose fiber instead of the water-repellent cellulose fiber, due to the properties of the water-repellent cellulose fiber. As a result, in a wet wipe formed from a nonwoven fabric comprising the water-repellent cellulose fiber, adjacent wet wipe portions are less likely to adhere each other and are more likely to separate, as compared to a wet wipe formed from a nonwoven fabric comprising hydrophilic cellulose fiber instead of the water-repellent cellulose fiber.
For example, when a wet wipe formed from the nonwoven fabric described above is folded along fold lines, among the wet wipe portions defined by the fold lines, adjacent wet wipe portions are less likely to adhere to each other and are more likely to separate, facilitating unfolding from the folded state. When a plurality of wet wipes formed from the nonwoven fabric described above are stacked, wet wipe portions adjacent in the stacking direction are less likely to adhere to each other and are more likely to separate. As a result, the multiple-wipe extraction tendency, in which a plurality of wet wipes, including the next wet wipe, are extracted at the same time when a single wet wipe is extracted, is less likely to occur. The multiple-wipe extraction tendency increases as the wet wipes hold a larger amount of chemical solution.
From the foregoing, excellent extractability of a wet wipe formed from the nonwoven fabric described above is achieved.
Furthermore, since the water-repellent cellulose fiber included in the nonwoven fabric is biodegradable, the nonwoven fabric, and ultimately, the wet wipe formed from the nonwoven fabric, is more likely to biodegrade than a nonwoven fabric comprising hydrophobic fiber, etc. instead of the water-repellent cellulose fiber, thereby reducing environmental impact.

[Aspect 2]

The nonwoven fabric according to Aspect 1, wherein the nonwoven fabric has a water absorptiveness according to the Klemm method of 60 to 110 mm.
Since the nonwoven fabric has a predetermined water absorptiveness according to the Klemm method, in a wet wipe formed from the nonwoven fabric, the wet wipe portions are less likely to adhere to each other and are more likely to separate, resulting in excellent extractability of the wet wipe formed from the nonwoven fabric.

[Aspect 3]

The nonwoven fabric according to Aspect 1 or 2, wherein the nonwoven fabric comprises 10 to 35 mass% of the water-repellent cellulose fiber.
Since the nonwoven fabric comprises a predetermined amount of the water-repellent cellulose fiber, in a wet wipe formed from the nonwoven fabric, the wet wipe portions are less likely to adhere together and are more likely to separate, resulting in excellent extractability of the wet wipe formed from the nonwoven fabric.

[Aspect 4]

The nonwoven fabric according to any one of Aspects 1 to 3, wherein the water-repellent cellulose fiber has a water swelling degree of 10 to 80 mass%.

In the nonwoven fabric described above, since the water-repellent cellulose fiber has a predetermined water swelling degree, in a wet wipe formed from the nonwoven fabric described above, the wet wipe portions are less likely to adhere to each other and are more likely to separate, resulting in excellent extractability of the wet wipe formed from the nonwoven fabric.

[Aspect 5]

The nonwoven fabric according to any one of Aspects 1 to 4, wherein the nonwoven fabric comprises a first surface layer and a second surface layer constituting surfaces of the nonwoven fabric, and an intermediate layer arranged between the first surface layer and the second surface layer,

> each of the first surface layer and the second surface layer comprises the water-repellent cellulose fiber, and
> the intermediate layer comprises a thermally fusible fiber.

Since the nonwoven fabric has a predetermined structure, and each of the first surface layer and second surface layer comprises the water-repellent cellulose fiber, in a wet wipe formed from the nonwoven fabric, the wet wipe portions are less likely to adhere to each other and are more likely to separate, resulting in excellent extractability of the wet wipe formed from the nonwoven fabric. Furthermore, since the intermediate layer comprises a thermally fusible fiber, a wet wipe formed from the nonwoven fabric has excellent wet strength, and a user is less likely to perceive stiffness derived from the thermally fusible fiber.

[Aspect 6]

The nonwoven fabric according to Aspect 5, wherein the thermally fusible fiber comprises a biodegradable thermally fusible fiber.

In the nonwoven fabric described above, since the thermally fusible fiber comprises a biodegradable thermally fusible fiber, the nonwoven fabric, and ultimately, the wet wipe, is more likely to be biodegradable.

[Aspect 7]

The nonwoven fabric according to Aspect 5 or 6, wherein each of the first surface layer and the second surface layer is composed of a cellulose fiber.

In the nonwoven fabric described above, since each of the first surface layer and the second surface layer is composed of a cellulose fiber, in a wet wipe formed from the nonwoven fabric, the chemical solution can more easily be retained in the first surface layer and second surface layer, resulting in the wet wipe being more likely to have excellent wiping properties.

[Aspect 8]

The nonwoven fabric according to any one of Aspects 5 to 7, wherein the intermediate layer further comprises a pulp fiber.

In the nonwoven fabric, since the intermediate layer further comprises a pulp fiber, in a wet wipe formed from the nonwoven fabric, the amount of chemical solution retained in the intermediate layer is relatively large, and the amount of chemical solution contained in the first surface layer and second surface layer is relatively small. As a result, the amount of chemical solution on the surfaces of the wet wipe portions (first surface layer and second surface layer) is relatively small, whereby the wet wipe portions are less likely to adhere to each other and are more likely to separate, resulting in excellent extractability of the wet wipe formed from the nonwoven fabric.

[Aspect 9]

A wet wipe, comprising a nonwoven fabric sheet composed of the nonwoven fabric according to any one of Aspects 1 to 8 and a chemical solution.

The wet wipe described above has the effects disclosed in Aspect 1.

[Aspect 10]

A package of wet wipes, including a package body comprising a top part having an opening, and a bottom part, and a plurality of wet wipes which are housed in an interior of the package body, wherein

at least a part of the plurality of wet wipes are composed of the wet wipe according to Aspect 9, and
the plurality of wet wipes are stacked in a direction from the bottom part toward the top part.

The package of wet wipes described above has the effects disclosed in Aspect 1.

[Aspect 11]

The package of wet wipes according to Aspect 10, wherein the plurality of wet wipes are arranged such that an end on a side of the top part of a bottom part side wet wipe positioned on a side of the bottom part and an end on a bottom part side of a top part side wet wipe adjacent to a top part side of the bottom part side wet wipe overlap each other such that the end on the top part side of the bottom part side wet wipe is positioned closer to the top part side than the end on the bottom part side of the top part side wet wipe.

The package of wet wipes described above tends to have low multiple-wipe extraction tendency and a predetermined extraction height. Thus, a user of wet wipes can easily extract the required number of wet wipes, and can easily extract the next wet wipe.

[Aspect 12]

Use of a water-repellent cellulose fiber for adjusting the extractability of a wet wipe.

In the use described above, the water-repellent cellulose fiber can adjust the extractability of a wet wipe.

The nonwoven fabric for a wet wipe (hereinafter sometimes simply referred to as "nonwoven fabric"), wet wipe, and package of wet wipes (hereinafter sometimes simply referred to as "package") according to the present disclosure will be described in detail below.

[Nonwoven Fabric]

The nonwoven fabric according to the present disclosure is a nonwoven fabric for a wet wipe, and comprises a water-repellent cellulose fiber. The water-repellent cellulose fiber is a cellulose fiber having water-repellency and exhibits water repellency, but once wetted, can retain a certain amount of water within the fiber and has a certain water swelling degree.

Thus, in a wet wipe formed from the nonwoven fabric comprising the water-repellent cellulose fiber described above, adjacent wet wipe portions are less likely adhere to each other and are more likely to separate, as compared to a wet wipe formed from a nonwoven fabric comprising hydrophobic fiber, etc., instead of the water-repellent cellulose fiber, and as compared to a wet wipe formed from a nonwoven fabric comprising hydrophilic cellulose fiber, etc., instead of the water-repellent cellulose fiber.

Furthermore, since the water-repellent cellulose fiber has a certain water swelling degree, a wet wipe formed from this nonwoven fabric is more likely to retain the chemical solution and has higher water retention, as compared to a nonwoven fabric comprising hydrophobic fiber instead of the water-repellent cellulose fiber. Furthermore, since the water-repellent cellulose fiber is biodegradable, the nonwoven fabric, and ultimately, the wet wipe formed from the nonwoven fabric, is more likely to biodegrade, reducing environmental impact.

Examples of the cellulose fiber constituting the water-repellent cellulose fiber include natural cellulose fibers, regenerated cellulose fibers, refined cellulose fibers, and semi-synthetic cellulose fibers.

Examples of natural cellulose fibers include plant fibers such as pulp fibers, seed fibers (for example, cotton fibers), bast fibers (for example, hemp), leaf fibers (for example, Manila hemp), and fruit fibers (for example, coir).

Examples of the pulp fibers include those which are well known as pulp fibers in the relevant technical field, for example, wood pulp fibers and non-wood pulp fibers. Examples of the wood pulp fibers include softwood pulp fibers and hardwood pulp fibers. Examples of the non-wood pulp fibers include straw pulp fibers, bagasse pulp fibers, reed pulp fibers, kenaf pulp fibers, mulberry pulp fibers, bamboo pulp fibers, hemp pulp fibers, and cotton pulp fibers (for example, cotton linter fibers).

Examples of the cotton fibers include Hirsutum cotton fibers (for example, upland cotton), Barbadense cotton fibers, Arboreum cotton fibers, and Herbaceum cotton fibers. TM

The cotton fiber can be an organic cotton fiber or Pre-Organic Cotton fiber.

Organic cotton fiber refers to cotton which has been certified by GOTS (Global Organic Textile Standard).

Examples of the regenerated cellulose fibers include rayon, for example, viscose rayon obtained from viscose, polynosic and modal, and cuprammonium rayon (also referred to as "cupra") obtained from a cuprammonium salt solution of cellulose.

The refined cellulose fiber may be lyocell, and specifically, fibers formed by dissolving pulp in an aqueous solution of N-methylmorpholine N-oxide to form a spinning dope, and extruding the spinning dope into a dilute solution of N-methylmorpholine N-oxide. The refined cellulose is commercially available under the trademark, for example, Tencel.

Examples of the semi-synthetic fibers include semi-synthetic cellulose fibers such as acetate fibers, for example, triacetate and diacetate fibers.

The water-repellent cellulose fiber can be formed by treating a cellulose fiber described above with a water-repellent agent, etc., in accordance with the methods described in, for example, Japanese Unexamined Patent Publication (Kokai) No. 2002-266241, Japanese Unexamined Patent Publication (Kokai) No. 2003-20570, Japanese Unexamined Patent Publication (Kokai) No. 2019-65443, Japanese Unexamined Patent Publication (Kokai) No. 2022-58301, etc.

As the water-repellent cellulose fiber, for example, Eco Repellas (trade name, water-repellent viscose rayon) manufactured by Daiwabo Rayon Co., Ltd. and Olea (trade name, water-repellent viscose rayon) manufactured by Kelheim Fibres GmbH are commercially available.

The water repellency of the water-repellent cellulose fiber described above can be evaluated as follows in accordance with "6(1) Ka: Sinking rate" in the "Standards for Medical Gauze and Medical Absorbent Cotton" attached to PFSB/ELD/OMDE Notification No. 0630001 "Standards for Medical Gauze and Medical Absorbent Cotton."

A 10 g sample, obtained by carding fibers constituting a nonwoven fabric or fibers collected from a wet wipe with a carding machine is placed evenly in a test basket having a diameter of 50 mm, a depth of 80 mm, and a weight of 3 g, made from copper wire having a diameter of 0.4 mm with a wire-to-wire distance of 20 mm. The basket is held horizontally and gently dropped into water having a depth of 200 mm at a temperature of 24 to 26°C from a height of 12 mm above the water surface. The sinking rate is determined as the time until the basket sinks below the water surface. For fibers that cannot be carded with a carding machine (for example, fibers having a short fiber length etc.), 10 g of the fibers can be placed in the test basket without carding. Alternatively, when the fiber product is in the form of a wet-laid or an air-laid nonwoven fabric, a 10 g sample of the nonwoven fabric cut into a 1 cm × 1 cm size can be placed in the test basket.

When the sinking rate is measured by the method described above, water-repellent cellulose fibers whose sample absorbs water but does not sink below the water surface after, for example, 1 minute, 5 minutes, or 10 minutes have elapsed, are preferably used. Water-repellent cellulose fibers whose sample does not sink and floats partially or completely on the water surface after 1 minute has elapsed are more preferably used.

The water-repellent cellulose fiber can be distinguished from a hydrophilic cellulose fiber by water swelling degree.

The water-repellent cellulose fiber preferably has a water swelling degree of 10 mass% or more, more preferably 15 mass% or more, and further preferably 20 mass% or more. The water-repellent cellulose fiber preferably has a water swelling degree of 80 mass% or less, more preferably 70 mass% or less, and further preferably 60 mass% or less. As a result, in a wet wipe formed from the nonwoven fabric, wet wipe portions are less likely to adhere to each other and are more likely to separate.

The hydrophilic cellulose fiber can have a water swelling degree higher than that of the water-repellent cellulose fiber. For example, the hydrophilic cellulose fiber preferably has a water swelling degree of greater than 60 mass%, more preferably greater than 70 mass%, further preferably greater than 80 mass%, even further preferably 85 mass% or more, and even further preferably 90 mass% or more. The hydrophilic cellulose fiber preferably has a water swelling degree of 300 mass% or less, more preferably 200 mass% or less, and further preferably 150 mass% or less.

The water-repellent cellulose fiber has a water swelling degree that is preferably at least 5 mass%, more preferably at least 10 mass%, further preferably at least 15 mass%, even further preferably at least 20 mass%, and even further preferably at least 25 mass% lower than that of a normal cellulose fiber which does not have water repellency, such as the hydrophilic cellulose fiber described below. As a result, in a wet wipe formed from the nonwoven fabric, wet wipe portions are less likely to adhere to each other and are more likely to separate.

In the present disclosure, the water swelling degree of a fiber is measured in accordance with "8.26 Water swelling degree" of "Test methods for man-made staple fibers" of JIS L1015: 2010, and this method is applied to fibers other than rayon and cupra.

The water-repellent cellulose fiber preferably has an average fiber length of 20 mm or more, more preferably 30 mm or more, and further preferably 35 mm or more. The water-repellent cellulose fiber preferably has an average fiber length of 80 mm or less, more preferably 60 mm or less, and further preferably 50 mm or less. As a result, the water-repellent cellulose fiber can more easily become entangled with other fibers, such as the water-repellent cellulose fiber and the optional hydrophilic cellulose fiber, whereby the strength of the nonwoven fabric, and ultimately, the wet wipe, tends to be increased.

The water-repellent cellulose fiber preferably has a fineness of 0.6 dtex or more, more preferably 1.0 dtex or more, and further preferably 1.4 dtex or more. The water-repellent cellulose fiber preferably has a fineness of 3.3 dtex or less, more preferably 2.5 dtex or less, and further preferably 2.0 dtex or less. As a result, fiber clumping in the nonwoven fabric comprising the water-repellent cellulose fiber can be suppressed while maintaining texture.

In the present disclosure, the average fiber length of fibers other than pulp fibers is measured in accordance with "A7.1.1 Method A (standard method) - measuring the length of individual fibers on a graduated glass plate" of "A7.1 Measurement of Fiber Length" of Annex A of JIS L 1015:2010.

The above method is a test method equivalent to ISO 6989 published in 1981.

In the present disclosure, the average fiber length of the pulp fiber means the weight-weighted average fiber length, and refers to the L(w) value measured with a Kajaani FiberLab fiber properties (off-line) manufactured by Metso Automation.

The nonwoven fabric preferably comprises the water-repellent cellulose fiber in a proportion of greater than 0 mass%, more preferably 4 mass% or more, further preferably 6 mass% or more, even further preferably 8 mass% or more, and even further preferably 10 mass% or more. The nonwoven fabric preferably comprises the water-repellent cellulose fiber in a proportion of 100 mass% or less, more preferably 70 mass% or less, further preferably 60 mass% or less, even further preferably 50 mass% or less, and even further preferably 35 mass% or less. As a result, the nonwoven fabric has a certain affinity for water, and in a wet wipe formed from the nonwoven fabric, wet wipe portions are less likely to adhere to each other and are more likely to separate.

The nonwoven fabric can further comprise a hydrophilic cellulose fiber. As a result, the nonwoven fabric itself is more likely to retain the chemical solution, whereby the wet wipe portions are less likely to adhere to each other and are more likely to separate. Furthermore, since the wet wipe formed from the nonwoven fabric is likely to retain the chemical solution, water retention is improved. Furthermore, since the hydrophilic cellulose fiber is biodegradable, the nonwoven fabric, and ultimately, the wet wipe formed from the nonwoven fabric, is more likely to biodegrade, reducing environmental impact.

From the above viewpoint, the nonwoven fabric preferably comprises the hydrophilic cellulose fiber in a proportion of 5 mass% or more, more preferably 10 mass% or more, further preferably 15 mass% or more, even further preferably 20 mass% or more, and even further preferably 25 mass% or more. The nonwoven fabric preferably comprises the hydrophilic cellulose fiber in a proportion of 90 mass% or less, more preferably 80 mass% or less, further preferably 70 mass% or less, even further preferably 60 mass% or less, and even further preferably 50 mass% or less.

Examples of the cellulose fiber constituting the hydrophilic cellulose fiber can include those described above in the section regarding the water-repellent cellulose fiber.

When the hydrophilic cellulose fiber is not a pulp fiber, the hydrophilic cellulose fiber preferably has an average fiber length of 20 mm or more, more preferably 30 mm or more, and further preferably 35 mm or more. When the hydrophilic cellulose fiber is not a pulp fiber, the hydrophilic cellulose fiber preferably has an average fiber length of 80 mm or less, more preferably 70 mm or less, and further preferably 60 mm or less. As a result, the hydrophilic cellulose fibers can more easily entangle with each other, which increases the strength of the nonwoven fabric, and ultimately, the wet wipe.

The hydrophilic cellulose fiber preferably has a fineness of 0.3 dtex or more, more preferably 0.4 dtex or more, and further preferably 0.6 dtex or more. The hydrophilic cellulose fiber preferably has a fineness of 4.5 dtex or less, more preferably 4.0 dtex or less, and further preferably 3.0 dtex or less. As a result, fiber clumping in the nonwoven fabric comprising the hydrophilic cellulose fiber can be suppressed while maintaining texture.

The nonwoven fabric can be composed of the water-repellent cellulose fiber and the hydrophilic cellulose fiber.

The nonwoven fabric may comprise, in addition to the water-repellent cellulose fiber and hydrophilic cellulose fiber, a thermally fusible fiber, a hydrophobic fiber, etc., which will be described later.

The nonwoven fabric can further comprise thermally fusible fibers intended to be thermally bonded in the nonwoven fabric. When the nonwoven fabric comprises thermally fusible fibers and thermally fusible is carried out between thermally fusible fibers, between thermally fusible fibers and cellulose fibers (water-repellent cellulose fibers and hydrophilic cellulose fibers), etc., the strength of the nonwoven fabric, and ultimately, the wet strength of the wet wipe, can be increased. When thermally fusible fibers are thermally bonded to each other, the strength of the nonwoven fabric, and ultimately, the wet strength of the wet wipe, can further be increased.

The thermally fusible fiber can be any fiber used in the relevant technical field, and specifically, may be a fiber comprising a low-melting-point thermoplastic resin and a high-melting-point thermoplastic resin, without limitation. In order to thermally bond the thermally fusible fibers together, it is preferable that the thermally fusible fiber comprise a low-melting-point thermoplastic resin, such as polyethylene resin or low-melting-point polypropylene, at least on the surface thereof. Examples of the thermally fusible fiber include mono-component fiber of polyethylene resin; mono-component fiber of polypropylene resin; core-sheath conjugate synthetic fiber having a polyethylene terephthalate resin core and a polyethylene resin sheath; core-sheath conjugate synthetic fiber having a polypropylene resin core and a polyethylene resin sheath; core-sheath conjugate synthetic fiber having a high-melting-point polypropylene resin core and a low-melting-point polypropylene resin sheath; side-by-side conjugate synthetic fiber consisting of polyethylene terephthalate resin and polyethylene resin; and side-by-side conjugate synthetic fiber consisting of polypropylene resin and polyethylene resin.

The thermally fusible fiber is preferably a biodegradable thermally fusible fiber. As a result, the nonwoven fabric, and ultimately, the wet wipe, is more likely to be biodegradable.

Examples of the low-melting thermoplastic resin constituting the biodegradable thermally fusible fiber include polybutylene succinate, poly(hydroxybutyrate/hydroxyhexanoate), polycaprolactone, poly(caprolactone/butylene succinate), poly(butylene succinate/adipate), poly(butylene succinate/carbonate), poly(butylene adipate/terephthalate), polyethylene succinate, and polylactic acid.

Examples of the high-melting thermoplastic resin constituting the biodegradable thermally fusible fiber include polylactic acid, polyhydroxybutyrate, polyglycolic acid, and cellulose acetate.

When the nonwoven fabric comprises the thermally fusible fiber, the nonwoven fabric preferably comprises the thermally fusible fiber in a proportion of greater than 0 mass%, more preferably 2 mass% or more, and further preferably 5 mass% or more of the thermally fusible fiber. When the nonwoven fabric comprises the thermally fusible fiber, the nonwoven fabric preferably comprises the thermally fusible fiber in a proportion of 30 mass% or less, more preferably 20 mass% or less, and further preferably 15 mass% or less. As a result, the wet wipe formed from the nonwoven fabric has excellent wet strength.

The nonwoven fabric can further comprise a hydrophobic fiber which is not intended to be thermally bonded in the nonwoven fabric. As a result, the wet wipe formed from the nonwoven fabric can have bulk, resistance to flattening, etc.

Examples of the hydrophobic fiber include those commonly used in the relevant technical field, preferably synthetic fiber. Examples of synthetic fiber include fiber comprising a single component, such as a mono-component fiber, or fiber comprising a plurality of components, such as a multi-component fiber.

Examples of the above components include polyolefin-based polymers such as polyethylene and polypropylene; polyester-based polymers such as terephthalate-based polymers such as polyethylene terephthalate (PET), polybutylene terephthalate, and polypentylene terephthalate; polyamide-based polymers such as nylon 6 and nylon 6,6; acrylic-based polymers; polyacrylonitrile-based polymers; and modified products thereof.

When the nonwoven fabric comprises the hydrophobic fiber, the nonwoven fabric preferably comprises the hydrophobic fiber in a proportion of greater than 0 mass%, more preferably 1 mass% or more, and further preferably 2 mass% or more. When the nonwoven fabric comprises the hydrophobic fiber, the nonwoven fabric preferably comprises the hydrophobic fiber in a proportion of 50 mass% or less, more preferably 40 mass% or less, and further preferably 35 mass% or less. As a result, the wet wipe formed from the nonwoven fabric can have bulk, resistance to flattening, etc.

The nonwoven fabric preferably has a basis weight of 20 g/m$^2$ or more, more preferably 30 g/m$^2$ or more, and further preferably 35 g/m$^2$ or more. The nonwoven fabric preferably has a basis weight of 100 g/m$^2$ or less, more preferably 90 g/m$^2$ or less, further preferably 80 g/m$^2$ or less, and even further preferably 70 g/m$^2$ or less. As a result, the extractability of the wet wipes formed from the nonwoven fabric and the strength thereof during wiping, etc., can be easily secured.

The nonwoven fabric can have a layered structure, for example, a two-layer structure, a three-layer structure, a four-layer structure, a five-layer structure, etc. (a structure of three or more layers may be referred to hereinafter as a "three or more layer structure").

When the nonwoven fabric has a three or more layer structure, the nonwoven fabric can be divided into a first surface layer and a second surface layer which constitute the surfaces of the nonwoven fabric, and one or more intermediate layers arranged between the first surface layer and the second surface layer.

When the nonwoven fabric has a three or more layer structure, the water-repellent cellulose fiber is preferably included in at least one of the first surface layer and the second surface layer, and more preferably in both the first surface layer and the second surface layer. As a result, in the wet wipe formed from the nonwoven fabric, the wet wipe portions are less likely to adhere to each other and are more likely to separate.

The intermediate layer can comprise the water-repellent cellulose fiber. However, from the viewpoint of facilitating separation of adjacent wet wipe portions, the intermediate layer need not comprise the water-repellent cellulose fiber.

When the nonwoven fabric has a three or more layer structure and the nonwoven fabric further comprises a hydrophilic cellulose fiber, the hydrophilic cellulose fiber can be included in any of the first surface layer, the intermediate layer, and the second surface layer, and is preferably included in all of the first surface layer, the intermediate layer, and the second surface layer. As a result, the wet wipe formed from the nonwoven fabric can be easily impregnated thoroughly with the chemical solution.

When the nonwoven fabric has a three or more layer structure and the nonwoven fabric further comprises the thermally fusible fiber, the thermally fusible fiber can be included in any of the first surface layer, intermediate layer, and second surface layer. The thermally fusible fiber can be included in at least the intermediate layer, or only the intermediate layer. As a result, the wet wipe formed from the nonwoven fabric has excellent wet strength, and a user is less likely to perceive stiffness derived from the thermally fusible fiber.

The thermally fusible fiber can also be included in the first surface layer and the second surface layer. As a result, the wet wipe formed from the nonwoven fabric is less susceptible to deformation, such as loss of shape or stretching, during wiping.

When the nonwoven fabric has a three or more layer structure, each of the first surface layer and the second surface layer is preferably composed of a cellulose fiber. As a result, the chemical solution is easily retained in the first surface layer and the second surface layer, and the wet wipe can easily have excellent wiping properties.

The cellulose fiber comprises the water-repellent cellulose fiber and the optional hydrophilic cellulose fiber.

When the nonwoven fabric has a three or more layer structure, the intermediate layer can further comprise a pulp fiber. As a result, the amount of chemical solution on the surfaces of the wet wipe portion (first surface layer and second surface layer) becomes relatively small, and the wet wipe portions are less likely to adhere to each other and are more likely to separate.

The nonwoven fabric according to the present disclosure preferably has a water absorptiveness according to the Klemm method of 60 mm or more, more preferably 65 mm or more, and further preferably 70 mm or more. The nonwoven fabric according to the present disclosure preferably has a water absorptiveness according to the Klemm method of 110 mm or less, more preferably 105 mm or less, and further preferably 100 mm or less. As a result, in the wet wipe formed from the nonwoven fabric, the wet wipe portions are less likely to adhere to each other and are more likely to separate.

In the present disclosure, the water absorptiveness according to the Klemm method is measured in accordance with JIS P8141:2004 "Paper and board - Determination of water absorptiveness (capillary rise) - Klemm method," and the differences from JIS P8141:2004 are as follows:

- Two types of test samples are used: one in which the conveying direction of the nonwoven fabric during production corresponds to the longitudinal direction of the test sample (hereinafter referred to as "MD test sample"), and one in which the conveying direction of the nonwoven fabric during production corresponds to the transverse direction of the test sample (hereinafter referred to as "CD test sample").
- The dimensions of the test samples are changed from "width 15±1 mm" to "width 25±1 mm."
- The time for measuring the height to which water rises is changed from "10 minutes±10 seconds" to "5 minutes±10 seconds."
- The average value of the Klemm water absorptiveness of the MD test sample and the CD test sample is adopted as the Klemm water absorptiveness.

The nonwoven fabric according to the present disclosure preferably has a water absorption capacity of 13.5 g/g or more, more preferably 14.0 g/g or more, further preferably 14.5 g/g or more, and even further preferably 15.0 g/g or more. The nonwoven fabric according to the present disclosure preferably has a water absorption capacity of 20.0 g/g or less, more preferably 19.0 g/g or less, further preferably 18.0 g/g or less, and even further preferably 17.0 g/g or less. As a result, the wet wipe formed from the nonwoven fabric has excellent wiping properties.

The nonwoven fabric according to the present disclosure preferably has a water retention capacity of 10.0 g/g or more, more preferably 10.3 g/g or more, further preferably 10.6 g/g or more, and even further preferably 10.9 g/g or more. The nonwoven fabric according to the present disclosure preferably has a water retention capacity of 16.0 g/g or less, more preferably 15.0 g/g or less, further preferably 14.0 g/g or less, and even further preferably 13.0 g/g or less. As a result, the wet wipe formed from the nonwoven fabric has excellent wiping properties.

In the present disclosure, the water absorption capacity and the water retention capacity are measured as follows.

(1) A 60 mm × 140 mm (conveying direction during production × direction perpendicular to the conveying direction) sample is prepared by cutting the nonwoven fabric, and the mass of the sample: $m_0$ (g) is measured.

(2) Ion-exchanged water is placed in a plastic container, the sample is placed on a wire mesh having a mass $m_1$ (g) which has been measured, and the sample together with the wire mesh is immersed in the ion-exchanged water.

(3) After 3 minutes of immersion, the sample together with the wire mesh is removed from the ion-exchanged water and allowed to stand for 5 minutes to drain.

(4) After allowing the sample to stand for 5 minutes, the total mass of the sample and the wire mesh: $m_2$ (g) is measured.

(5) The sample containing the ion-exchanged water is gripped with tweezers without applying pressure, and is allowed to stand on a metal base having a size of 60 mm × 140 mm or more such that the sample has no wrinkles.

(6) An 840 g weight is placed on the sample so that the load is evenly distributed over the entire sample, and allowed to stand for 3 minutes.

(7) After the sample has been allowed to stand for 3 minutes, the mass of the sample: $m_3$ (g) is measured.

(8) The water absorption capacity: A (g/g) is calculated using the following formula:

$$A\ (g/g) = (m_2 - m_1 - m_0)/m_0$$

(9) Water retention capacity: R (g/g) is calculated using the following formula:

$$R\ (g/g) = (m_3 - m_0)/m_0$$

The water absorption capacity and water retention capacity are measured in a thermostatic chamber at 25°C ±5°C.

The nonwoven fabric may be any nonwoven fabric (for example, a spunlace nonwoven fabric, an air-through nonwoven fabric, a spunbond nonwoven fabric, a point-bond nonwoven fabric, etc.) produced by a known method such as a dry method, a wet method, a water jet method, a spunbond method, an airlaid method, a needle punch method, or a felt method, and is preferably a spunlace nonwoven fabric having a high wet strength.

The nonwoven fabric can be produced by a method which is known in the relevant technical field. When the nonwoven fabric is a spunlace nonwoven fabric, for example, a dry spunlace nonwoven fabric, it can be produced by, for example, a dry spunlace method in which a single-layer or multi-layer web constituting the nonwoven fabric, formed by a dry process, is arranged on a support and a high-pressure water jet is sprayed onto the web from a nozzle.

When the nonwoven fabric comprises the thermally fusible fiber, a high-pressure water jet may be sprayed onto the web, and the web (nonwoven fabric) may then be heated to a temperature above the melting point of the low-melting thermoplastic resin constituting the thermally fusible fiber to thermally bond the thermally fusible fiber.

[Wet Wipe and Package of Wet Wipes]

The wet wipe according to the present disclosure comprises a nonwoven fabric sheet composed of the nonwoven fabric described above and a chemical solution.

The nonwoven fabric sheet is formed by cutting the nonwoven fabric described above to a desired size.

The chemical solution can be any chemical solution used in the relevant technical field without limitation.

The chemical solution can comprise, for example, an antibacterial agent, a surfactant, water, a hydrophilic organic solvent (for example, ethanol), etc.

In the wet wipe described above, the ratio (impregnation rate) of the chemical solution with which the nonwoven fabric is impregnated can be any ratio commonly used in the relevant technical field, and the impregnation rate (mass%) [=100×(mass of chemical solution)/(mass of nonwoven fabric)] is, for example, 100 mass% or more, more preferably 200 mass% or more, and further preferably 250 mass% or more. The ratio (impregnation rate) is preferably 700 mass% or less, more preferably 600 mass% or less, further preferably 550 mass% or less, and even further preferably 500 mass% or less.

A package of wet wipes comprises the wet wipe and a package body, and the wet wipe is housed in a package body. Examples of the package body include a packaging sheet and a packaging container.

When the package body is a packaging sheet, in the package of wet wipes, one or more wet wipes are housed in the packaging sheet. When the package comprises one wet wipe, the one wet wipe is folded. When the package comprises a plurality of wet wipes, the plurality of wet wipes may or may not be folded.

When the package body is a packaging container, in the package of wet wipes, a plurality of wet wipes are housed in the packaging container.

The packaging container may have a top part having an opening and a bottom part, and the plurality of wet wipes can be stacked in a direction from the bottom part toward the top part.

Among the plurality of wet wipes, a portion of a bottom part side wet wipe, positioned on the bottom part side, and a portion of a top part side wet wipe, adjacent to the top part side of the bottom part side wet wipe, can be arranged so as to overlap each other such that the portion of the bottom part side wet wipe is positioned closer to the top part side than the portion of the top part side wet wipe.

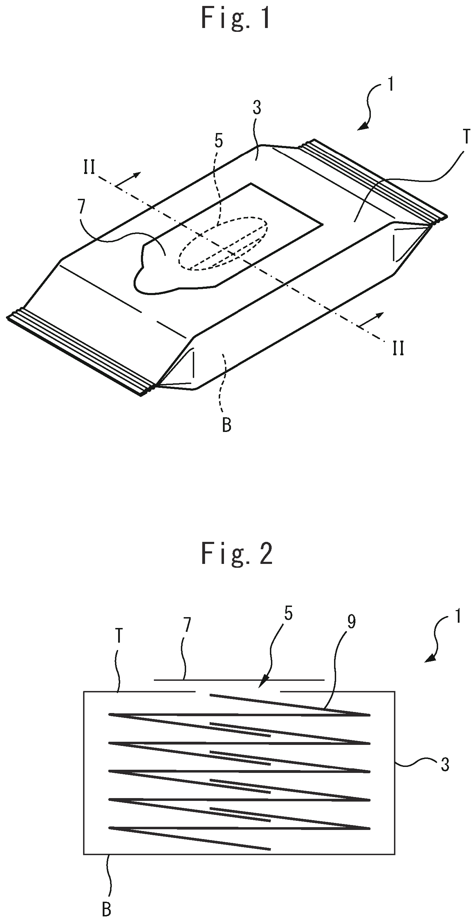

FIG. 1 is a perspective view of a package of wet wipes 1 according to an embodiment of the present disclosure (hereinafter referred to as "the first embodiment"), and FIG. 2 is an end view of the package of wet wipes 1 taken along line II-II shown in FIG. 1.

The package of wet wipes 1 shown in FIG. 1 is a pop-up type package of wet wipes in which a plurality of wet wipes (not illustrated) are housed inside a package body 3 having a top part T with an opening 5 and a bottom part B so that the plurality of wet wipes can be extracted sequentially.

The package body 3 has a seal member 7 for covering the opening 5.

The package body 3 is bag-shaped, and when the opening 5 is sealed with the seal member 7, the wet wipes inside the package body 3 can be sealed, preventing evaporation of the chemical. Each wet wipe can be extracted through the opening 5. The seal member 7 has an adhesive applied to the surface which contacts the package body 3 so that the wet wipes inside the package body 3 can be repeatedly sealed.

As shown in FIG. 2, inside the package body 3, the plurality of wet wipes 9 are stacked in a direction from the top part T to the bottom part B. Each of the plurality of wet wipes 9 has an outward tri-fold (substantially Z-shaped) structure formed along a first fold line and a second fold line. The plurality of wet wipes 9 are stacked so that adjacent wet wipes 9 in the thickness direction (the direction from the top part T to the bottom part B) face the same direction (so that the first fold lines overlap each other in the thickness direction, and so that the second fold lines overlap each other in the thickness direction).

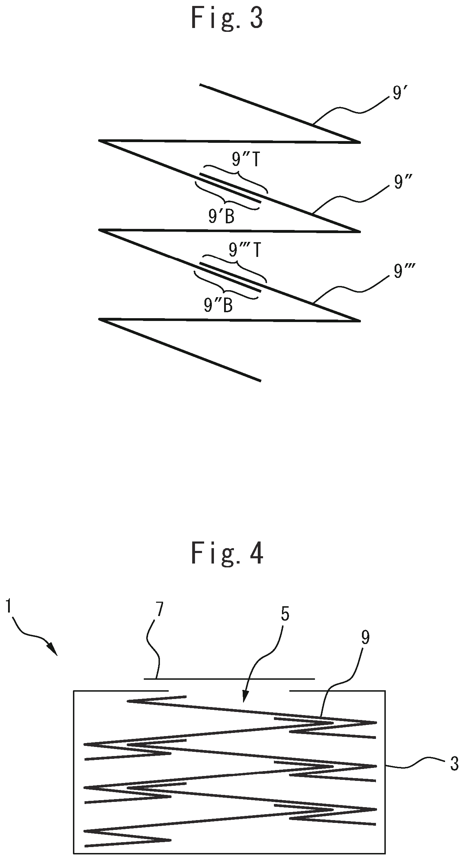

FIG. 3 is a view detailing the plurality of wet wipes 9 housed in the package of wet wipes 1 shown in FIG. 2.

Wet wipe 9', wet wipe 9", and wet wipe 9‴ are stacked in order in the direction from the top part T to the bottom part B. Regarding the relationship between the wet wipe 9' and the wet wipe 9", top part side end 9"T of the wet wipe 9" (bottom part side wet wipe), positioned on the bottom part B side, and bottom part side end 9'B of the wet wipe 9' (top part side wet wipe), adjacent to the top part T side thereof, overlap each other so that the top part side end 9"T of the wet wipe 9" (bottom part side wet wipe) is positioned closer to the top part T side than the bottom part side end 9'B of the wet wipe 9' (top part side wet wipe).

Likewise, in FIG. 3, regarding the relationship between the wet wipe 9" and the wet wipe 9‴, top part side end 9‴T of the wet wipe 9‴ (bottom part side wet wipe), positioned on the bottom part B side, and bottom part side end 9"B of the wet wipe 9" (top part side wet wipe), adjacent to the top part T side thereof, overlap each other so that the top part side end 9‴T of the wet wipe 9‴ (bottom part side wet wipe) is positioned closer to the top part T side than the bottom part side end 9"B of the wet wipe 9" (top part side wet wipe).

By stacking the plurality of wet wipes 9 as shown in FIGS. 2 and 3, when the wet wipe 9' is extracted, a portion of the wet wipe 9" (primarily, 9"T) is pulled out and protrudes from the package body 3, making it easier to extract the wet wipe 9" upon subsequent use.

When the bottom part end 9'B of the wet wipe 9' and the top part end 9"T of the wet wipe 9" are connected by a water film of chemical solution formed between the wet wipes, the multiple-wipe extraction tendency, in which when the wet wipe 9' is extracted, the entire wet wipe 9" is extracted together, may occur.

In the package of wet wipes 1 according to the first embodiment, the wet wipe 9 is a specific wet wipe, and thus, excellent single-wipe extractability is achieved.

FIG. 4 is an end view of the package of wet wipes 1 according to another embodiment of the present disclosure (hereinafter referred to as "the second embodiment"), corresponding to the end view taken along line II-II of FIG. 1. In FIG. 4, each of the plurality of wet wipes 9 has a four-fold (substantially Σ-shaped) structure formed along three fold lines. The plurality of wet wipes 9 are stacked such that the Σ shapes of adjacent wet wipes 9 in the thickness direction (the direction from the top part T to the bottom part B) face in opposite directions.

In the package of wet wipes 1 according to the second embodiment, in the same manner as the first embodiment, a top part side end (not illustrated) of a bottom part side wet wipe (not illustrated), positioned on the bottom part side, and a bottom part side end (not illustrated) of a top part side wet wipe (not illustrated), adjacent to the top part side of the bottom part side wet wipe, overlap each other so that the top part side end of the bottom part side wet wipe is positioned closer to the top part side than the bottom part side end of the top part side wet wipe.

In the package of wet wipes 1 according to the second embodiment, the wet wipe 9 is a specific wet wipe, and thus, excellent single-wipe extractability is achieved.

EXAMPLES

The present disclosure will be described below using Examples, but the present disclosure is not limited to these examples.

[Production Example 1]

Nonwoven fabric No. 1, which comprised a first surface layer, a first intermediate layer, a second intermediate layer, and a second surface layer, in that order, was formed by a dry spunlace method.

Each of the first surface layer and the second surface layer comprised 30 mass% of water-repellent rayon fiber (Eco Repellas, manufactured by Daiwabo Rayon Co., Ltd., fineness: 1.7 dtex, average fiber length: 40 mm) and 70 mass% of hydrophilic rayon fiber (CORONA, manufactured by Daiwabo Rayon Co., Ltd., fineness: 1.44 dtex, average fiber length: 44 mm).

The first intermediate layer comprised 85 mass% of pulp fiber (softwood pulp fiber) and 15 mass% of thermally fusible fiber (NBF(KK)-PL, manufactured by Daiwabo Co., Ltd.; conjugate fiber with a polylactic acid core and a polybutylene succinate sheath; fineness: 2.40 dtex, average fiber length: 5.0 mm). The second intermediate layer comprised 100 mass% of hydrophilic rayon fiber (CORONA, manufactured by Daiwabo Rayon Co., Ltd.; fineness:

1.44 dtex, average fiber length: 44 mm).

Nonwoven fabric No. 1 was cut to a size of 200×140 mm (extraction direction×width direction), 50 sheets of the cut nonwoven fabric No. 1 were stacked as illustrated in the second embodiment, the 50 stacked sheets were wrapped in a package body, and the package body was then filled with a chemical solution equivalent to 300 mass% of the 50 stacked sheets, thereby forming package of wet wipes No. 1.

[Production Example 2]

Nonwoven fabric No. 2 and package of wet wipes No. 2 were formed in the same manner as in Production Example 1, except that the compositions of the first surface layer and the second surface layer were changed as shown in Table 1.

[Production Example 3]

Nonwoven fabric No. 3 and package of wet wipes No. 3 were formed in the same manner as in Production Example 1, except that the compositions of the first surface layer and the second surface layer were changed as shown in Table 1.

[Reference Production Example 1]

Nonwoven fabric No. 4 and package of wet wipes No. 4 were formed in the same manner as in Production Example 1, except that the compositions of the first surface layer and the second surface layer were changed as shown in Table 1.

[Comparative Production Example 1]

Nonwoven fabric No. 5 and package of wet wipes No. 5 were formed in the same manner as in Production Example 1, except that the compositions of the first surface layer and the second surface layer were changed as shown in Table 2, and specifically, the "water-repellent rayon fiber" of the first surface layer and the second surface layer was changed to a "hydrophobic fiber (polyethylene terephthalate fiber, fineness: 1.45 dtex, average fiber length: 38 mm)."

[Table 1]

Table 1

| Prod Ex No. | | Prod Ex 1 | | Prod Ex 2 | | Prod Ex 3 | | Ref Prod Ex 1 | |
|---|---|---|---|---|---|---|---|---|---|
| Nonwoven fabric No. | | No. 1 | | No. 2 | | No. 3 | | No. 4 | |
| Layer | Fiber | Proportion (mass%) | Basis wt (g/m$^2$) | Proportion (mass%) | Basis wt (g/m$^2$) | Proportion (mass%) | Basis wt (g/m$^2$) | Proportion (mass%) | Basis wt (g/m$^2$) |
| First surface layer | Water-repellent rayon fiber | 30 | 14 | 50 | 14 | 70 | 14 | 0 | 14 |
| First surface layer | Hydrophilic rayon fiber | 70 | | 50 | | 30 | | 100 | |
| First intermediate layer | Pulp fiber | 85 | 17 | 85 | 17 | 85 | 17 | 85 | 17 |
| First inter-mediate layer | Thermally fusible fiber | 15 | | 15 | 45 | 15 | 45 | 15 | 45 |
| Second inter-mediate layer | Hydrophilic rayon fiber | 100 | 7 | 100 | 7 | 100 | 7 | 100 | 7 |
| Second surface layer | Water-repellent rayon fiber | 30 | 7 | 50 | 7 | 70 | 7 | 0 | 7 |
| Second sur-face layer | Hydrophilic rayon fiber | 70 | | 50 | | 30 | | 100 | |
| Fiber structure | Water-repellent rayon fiber (mass%) | 14.0 | | 23.3 | | 32.7 | | 0.0 | |
| Fiber structure | Hydrophilic rayon fiber (mass%) | 48.2 | | 38.9 | | 29.6 | | 62.2 | |
| Fiber structure | Pulp fiber (mass%) | 32.1 | | 32.1 | | 32.1 | | 32.1 | |
| Fiber structure | Thermally fusible fiber (mass%) | 5.7 | | 5.7 | | 5.7 | | 5.7 | |

EP 4 756 090 A1

[Table 2]

Table 2

| Prod Ex No. | | Comp Prod Ex 1 | | |
|---|---|---|---|---|
| Nonwoven fabric No. | | No. 5 | | |
| Layer | Fiber | Proportion (mass%) | Basis wt (g/m$^2$) | |
| First surface layer | Water-repellent rayon fiber | 70 | 14 | 45 |
| | Hydrophilic rayon fiber | 30 | | |
| First intermediate layer | Pulp fiber | 85 | 17 | |
| | Thermally fusible fiber | 15 | | |
| Second intermediate layer | Hydrophilic rayon fiber | 100 | 7 | |
| Second surface layer | Water-repellent rayon fiber | 70 | 7 | |
| | Hydrophilic rayon fiber | 30 | | |
| Fiber structure | Water-repellent rayon fiber (mass%) | 32.7 | | |
| | Hydrophilic rayon fiber (mass%) | 29.6 | | |
| | Pulp fiber (mass%) | 32.1 | | |
| | Thermally fusible fiber (mass%) | 5.7 | | |

[Examples 1 to 3 and Comparative Examples 1 and 2]

[Water Absorptiveness According to Klemm Method]

The water absorptiveness according to the Klemm method, water absorption capacity, and water retention capacity of nonwoven fabric No. 1 to nonwoven fabric No. 5 were measured in accordance with the methods described herein. The results are shown in Table 3.

For each of package of wet wipes No. 1 to package of wet wipes No. 5, multiple-wipe extractability and extraction height were evaluated. The test methods are as follows. The results are shown in Table 3.

[Multiple-Wipe Extractability]

Wet wipes were extracted one by one from a package, and when the nth wet wipe (1, 2, ..., n, ..., 50, from the top) was extracted, the number of wet wipes that were pulled along with the nth wet wipe (wet wipe directly extracted) and were extracted entirely as a result (wet wipes indirectly "entirely" extracted) were counted.

For example, when the nth wet wipe was extracted, if the (n+1)th wet wipe, one wipe therebelow, was pulled along with the nth wet wipe and extracted in its entirety, the wet wipe indirectly "entirely" extracted was counted as one.

When the nth wet wipe was extracted, if the (n+1)th wet wipe, one wipe therebelow, was pulled along with the nth wet wipe and extracted in its entirety, and the (n+2)th wet wipe, two wipes therebelow, was pulled along with the (n+1)th wet wipe and extracted in its entirety, the wet wipe indirectly "entirely" extracted was counted as two.

The experiment was continued until all 50 wet wipes were extracted, and the total number (m) of wet wipes that were indirectly "entirely" extracted was counted and designated as the multiple-wipe extractability (wipes).

[Extraction Height]

Wet wipes were extracted one by one from a package, and when the nth wet wipe (1, 2, ..., n, ..., 50 from the top) was extracted, the extracted length (the length from the edge of the wet wipe to the opening) of the wet wipe a "portion" of which was extracted (the wet wipe a "portion" of which was indirectly extracted) by being pulled along with the nth wet wipe (the wet wipe directly extracted), was measured, and the average of all values was taken as the extraction height.

During measurement of extraction height, a wet wipe which was indirectly "entirely" extracted was ignored.

[Table 3]

Table 3

| | Ex 1 | Ex 2 | Ex 3 | Comp Ex 1 | Comp Ex 2 |
|---|---|---|---|---|---|
| Nonwoven fabric No. | No. 1 | No. 2 | No. 3 | No. 4 | No. 5 |
| Package No. | No. 1 | No. 2 | No. 3 | No. 4 | No. 5 |
| Water-repellent rayon fiber Hydrophobic fiber (mass%) | 14.0 | 23.3 | 32.7 | 0.0 | 32.7 |
| Hydrophilic rayon fiber (mass%) | 48.2 | 38.9 | 29.6 | 62.2 | 29.6 |
| Pulp fiber (mass%) | 32.1 | 32.1 | 32.1 | 32.1 | 32.1 |
| Thermally fusible fiber (mass%) | 5.7 | 5.7 | 5.7 | 5.7 | 5.7 |
| Water absorptiveness/mm | 93 | 83 | 64 | 120 | 93 |
| Multiple-wipe extractability /wipes | 2.7 | 2.7 | 2.3 | 5.7 | 5.3 |
| Extraction height/mm | 80 | 65 | 75 | 110 | 101 |
| Water absorption capacity (g/g) | 16 | 16 | 16 | 13.4 | 10.8 |
| Water retention capacity (g/g) | 10 | 11 | 11 | 9.7 | 8.4 |

REFERENCE SIGNS LIST

1    package of wet wipes

3    package body

5    opening

7    seal member

9    wet wipe

**Claims**

1. A nonwoven fabric for a wet wipe, wherein
the nonwoven fabric comprises a water-repellent cellulose fiber.

2. The nonwoven fabric according to claim 1, wherein the nonwoven fabric has a water absorptiveness according to the Klemm method of 60 to 110 mm.

3. The nonwoven fabric according to claim 1 or 2, wherein the nonwoven fabric comprises 10 to 35 mass% of the water-repellent cellulose fiber.

4. The nonwoven fabric according to any one of claims 1 to 3, wherein the water-repellent cellulose fiber has a water swelling degree of 10 to 80 mass%.

5. The nonwoven fabric according to any one of claims 1 to 4, wherein the nonwoven fabric comprises a first surface layer and a second surface layer constituting surfaces of the nonwoven fabric, and an intermediate layer arranged between the first surface layer and the second surface layer,

each of the first surface layer and the second surface layer comprises the water-repellent cellulose fiber, and
the intermediate layer comprises a thermally fusible fiber.

6. The nonwoven fabric according to claim 5, wherein the thermally fusible fiber comprises a biodegradable thermally fusible fiber.

7. The nonwoven fabric according to claim 5 or 6, wherein each of the first surface layer and the second surface layer is composed of a cellulose fiber.

8. The nonwoven fabric according to any one of claims 5 to 7, wherein the intermediate layer further comprises a pulp fiber.

9. A wet wipe, comprising a nonwoven fabric sheet composed of the nonwoven fabric according to any one of claims 1 to 8, and a chemical solution.

10. A package of wet wipes including a package body comprising a top part having an opening, and a bottom part, and a plurality of wet wipes which are housed in an interior of the package body, wherein

at least a part of the plurality of wet wipes are composed of the wet wipe according to claim 9, and
the plurality of wet wipes are stacked in a direction from the bottom part toward the top part.

11. The package of wet wipes according to claim 10, wherein the plurality of wet wipes are arranged such that an end on a side of the top part of a bottom part side wet wipe positioned on a side of the bottom part and an end on a bottom part side of a top part side wet wipe adjacent to a top part side of the bottom part side wet wipe overlap each other such that the end on the top part side of the bottom part side wet wipe is positioned closer to the top part side than the end on the bottom part side of the top part side wet wipe.

12. Use of a water-repellent cellulose fiber for adjusting extractability of a wet wipe.

# Fig. 1

# Fig. 2

Fig. 3

9′

9″T

9′B

9″

9‴T

9″B

9‴

Fig. 4

1

7

5

9

3

## INTERNATIONAL SEARCH REPORT

| International application No. |
| --- |
| **PCT/JP2024/030175** |

| A. | CLASSIFICATION OF SUBJECT MATTER |
| --- | --- |

*D04H 1/425*(2012.01)i; *A47K 7/00*(2006.01)i; *A47L 13/17*(2006.01)i
FI:   D04H1/425; A47K7/00 E; A47K7/00 H; A47L13/17 A

According to International Patent Classification (IPC) or to both national classification and IPC

| B. | FIELDS SEARCHED |
| --- | --- |

Minimum documentation searched (classification system followed by classification symbols)

D04H1/425; A47K7/00; A47L13/17

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2023-101408 A (DAIWA SPINNING CO., LTD.) 20 July 2023 (2023-07-20) | 1-7, 9 |
|   | claims, examples |   |
| Y | claims, examples | 10, 11 |
| A | claims, examples | 8, 12 |
| X | WO 2014/026207 A1 (LENZING AG) 20 February 2014 (2014-02-20) | 1-4, 9, 10 |
|   | claims, p. 7 |   |
| Y | claims, p. 7 | 11 |
| A | claims, p. 7 | 5-8, 12 |
| X | JP 2015-507977 A (LENZING AG) 16 March 2015 (2015-03-16) | 1, 9 |
|   | claims, examples |   |
| X | US 2005/0245159 A1 (CHMIELEWSKI, Harry J.) 03 November 2005 (2005-11-03) | 1, 9 |
|   | claims |   |

| ✓ Further documents are listed in the continuation of Box C. | ✓ See patent family annex. |
| --- | --- |

| \* | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **18 October 2024** | **29 October 2024** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan |   |
|   | Telephone No. |

Form PCT/ISA/210 (second sheet) (July 2022)

**INTERNATIONAL SEARCH REPORT**

| International application No. |
|---|
| **PCT/JP2024/030175** |

| C. | DOCUMENTS CONSIDERED TO BE RELEVANT | |
|---|---|---|
| Category* | Citation of document, with indication, where appropriate, of the relevant passages | Relevant to claim No. |
| Y | JP 2022-092982 A (UNI-CHARM CO., LTD.) 23 June 2022 (2022-06-23)<br>claim 12, fig. 1-4 | 10, 11 |

Form PCT/ISA/210 (second sheet) (July 2022)

## INTERNATIONAL SEARCH REPORT
### Information on patent family members

International application No.

**PCT/JP2024/030175**

| Patent document cited in search report | | | Publication date (day/month/year) | Patent family member(s) | | | Publication date (day/month/year) |
|---|---|---|---|---|---|---|---|
| JP | 2023-101408 | A | 20 July 2023 | (Family: none) | | | |
| WO | 2014/026207 | A1 | 20 February 2014 | AT | 513349 | A | |
| | | | | TW | 201422866 | A | |
| JP | 2015-507977 | A | 16 March 2015 | US | 2015/0044926 | A1 | |
| | | | | claims, examples | | | |
| | | | | WO | 2013/126934 | A1 | |
| | | | | EP | 2819712 | A1 | |
| | | | | AT | 512621 | A | |
| | | | | TW | 201406354 | A | |
| | | | | CN | 104244997 | A | |
| | | | | SI | 2819712 | T | |
| | | | | TR | 201909346 | T | |
| | | | | PL | 2819712 | T | |
| | | | | ES | 2733053 | T | |
| US | 2005/0245159 | A1 | 03 November 2005 | (Family: none) | | | |
| JP | 2022-092982 | A | 23 June 2022 | WO | 2022/124031 | A1 | |
| | | | | claim 12, fig. 1-4 | | | |
| | | | | CN | 116669691 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014094188 A
- JP 2022092982 A
- JP 2002266241 A
- JP 2003020570 A
- JP 2019065443 A
- JP 2022058301 A